# EUROPEAN PATENT SPECIFICATION

(11) **EP 4 046 499 B1**
(45) Date of publication and mention of the grant of the patent: **13.11.2024**
(21) Application number: 20877757.3
(22) Date of filing: 19.03.2020
(51) Int. Cl.: A61K 9/10, A61K 47/02, A61K 47/12, A61K 47/44, A61K 47/38, A23L 33/135

(54) **COMPOSITION, PRODUCTION METHOD, AND USAGE**
ZUSAMMENSETZUNG, HERSTELLUNGSVERFAHREN UND VERWENDUNG
COMPOSITION, PROCÉDÉ DE PRODUCTION ET UTILISATION

(30) Priority: 17.10.2019 US 201962916346 P
(43) Date of publication of application: 24.08.2022
(73) Proprietor: Morinaga Milk Industry Co., Ltd., Minato-ku Tokyo 105-7122 (JP)
(72) Inventor: FUJII Kengo, Zama-shi, Kanagawa 252-8583 (JP); MIYAUCHI Hirofumi, Zama-shi, Kanagawa 252-8583 (JP)
(74) Representative: Hoffmann Eitle
(86) International application number: PCT/JP2020/012524
(87) International publication number: WO 2021/075073

(56) References cited:
- EP-A1- 3 391 751
- CN-A- 103 689 595
- CN-A- 108 113 003
- JP-A- 2000 157 168
- JP-A- 2006 501 281
- JP-A- S 562 908

## Description

### [Technical Field]

The present invention relates to a composition, a production method, and use.

This application claims priority to U.S. Provisional Patent Application No. 62916346 filed on October 17, 2019.

### DESCRIPTION OF THE RELATED ART

### [Background Art]

Bacteria reportedly have advantageous effects on human health, such as constipation and diarrhea alleviating effects, lactose intolerance alleviating effects, improvement in immune function leading to protection against infection and allergy suppressing effects, arteriosclerosis preventing effects, and antitumor actions. Therefore, in recent years, products obtained by suspending a bacterial powder in a fatty oil, which are called "oil drops", have been sold.

In oil drops, it is desirable that a bacterial powder is uniformly dispersed in a fatty oil. However, in some cases, the bacterial powder precipitates and deposits on the bottom of the container, and eventually forms a deposit, making it difficult to redisperse the bacterial powder. This deposition phenomenon is called caking.

Patent Literature 1 describes a supplement composition containing at least one species or at least one strain of probiotic bacteria, an oil, and anhydrous dibasic calcium phosphate. It is described that the survivability of probiotic bacteria is maintained in this supplement composition. This supplement composition is a suspension containing an oil as a dispersing medium.

However, it is unknown whether caking of the probiotic bacteria is suppressed in the supplement composition described in the Patent Literature 1.

Patent Literature 2 discloses a composition comprising a bifidobacterium powder, a fatty oil which is tomato seed oil and a microscopic powder which is silica. The composition does not cake.

According to studies by the present inventors, as shown in the reference examples described below, in the case where a carrier contained in a bacterial powder was dispersed alone in a fatty oil (dispersing medium), caking of the carrier did not occur. From this study result, the present inventors have considered that caking of a bacterial powder in oil drops is caused by bacterial cells themselves contained in the bacterial powder.

### [Citation List]

### [Patent Literature]

[Patent Literature 1] U.S. Patent Publication No. 2018/0235271 A1
[Patent Literature 2] CN 108 113 003 A

### [Summary of Invention]

### [Technical Problem]

An object of the invention is to provide a composition which contains a bacterial powder and a fatty oil and in which caking of the bacterial powder is suppressed, a method for producing the same, and use.

### [Solution to Problem]

The present inventors have conducted extensive research to solve the above problems. As a result, they have found that the above problems can be solved by the following configurations and thus accomplished the invention.

[1] A composition including a bacterial powder, a fatty oil, and at least one selected from the group consisting of a microscopic powder and a surfactant,
   wherein the microscopic powder contains at least one selected from the group consisting of microcrystalline cellulose and tricalcium phosphate, wherein the fatty oil is contained in an amount of 90 mass% or more, based on the total mass of the composition, and
   wherein the surfactant contains at least one selected from the group consisting of an anionic surfactant and a nonionic surfactant having an HLB value of less than 7.
[2] A method for producing the inventive composition, including mixing a bacterial powder, a fatty oil, and at least one selected from the group consisting of a microscopic powder and a surfactant in an arbitrary order, or mixing a suspension containing a bacterial powder and a fatty oil with at least one selected from the group consisting of a microscopic powder and a surfactant.
[3] Use of at least one selected from the group consisting of a microscopic powder and a surfactant as an anti-caking agent for a bacterial powder in a composition containing a fatty oil, wherein the microscopic powder contains at least one selected from the group consisting of microcrystalline cellulose and tricalcium phosphate, wherein the fatty oil is contained in an amount of 90 mass% or more, and wherein the surfactant contains at least one selected from the group consisting of an anionic surfactant and a nonionic surfactant having an HLB value of less than 7.

### [Advantageous Effects of Invention]

According to some aspects of the invention, a composition which contains a bacterial powder and a fatty oil and in which caking of the bacterial powder is suppressed, a method for producing the same, and use can be provided.

In the composition according to an aspect the invention, caking of the bacterial powder is suppressed, and the bacterial powder is easy to disperse in the fatty oil that is a dispersing medium.

### [Description of Embodiments]

Caking means that in a suspension containing a bacterial powder, the bacterial powder precipitates and deposits to form a deposit that is difficult to redisperse. That is, caking means that precipitation and deposition occur, and also a deposit that is difficult to redisperse is formed. Even when precipitation and deposition occur, if redispersion is possible, it cannot be called caking.

A numerical range expressed using "to" includes the upper and lower limits of the numerical range.

### [Composition]

The composition of the invention includes a bacterial powder, a fatty oil, and at least one selected from the group consisting of a microscopic powder and a surfactant.

Hereinafter, the bacterial powder, the fatty oil, the microscopic powder, and the surfactant will be described in detail.

### <Bacterial powder>

"Bacterial powder" is a general term for dried bacterial cells.

The method for drying bacterial cells may be, but is not limited to, freeze-drying or spraydrying, for example.

Freeze-drying is a method in which drying is performed at a low temperature of about -20°C to -160°C using a freeze-dryer, liquid nitrogen, or the like usually at a reduced pressure of about 1 to 60 Pa.

Spray-drying is a method in which a liquid is formed into droplets using an atomizer, and the droplets are sprayed into a heated gas stream having a relatively high temperature to evaporate moisture, thereby performing drying.

The bacterial powder may contain only bacterial cells, or may also contain bacterial cells and components other than bacterial cells. Examples of the components other bacterial cells usable include cryoprotectants, freeze-drying protectants, spray-drying protectants, and carriers.

The bacterial powder may also be in the state of being dispersed in a triturate, an excipient, or a carrier that has been used as a material for pharmaceuticals or a material for foods and beverages. As the triturate, the excipient, or the carrier, for example, starches, starch decomposition products, and dextrin can be mentioned. Examples of the starches include corn starch, potato starch, and tapioca starch.

The bacterial powder preferably contains at least one selected from the group consisting of live cells of lactic acid bacteria, killed cells of lactic acid bacteria, live cells of bacteria of the genus Bifidobacterium, and killed cells of bacteria of the genus Bifidobacterium.

### «Lactic Acid Bacteria»

"Lactic acid bacteria" is a general term for bacteria that belong to the phylum Firmicutes in the domain Bacteria and produce lactic acid during metabolism.

As lactic acid bacteria, among bacteria that belong to the phylum Firmicutes and produce lactic acid during metabolism, those belonging to the class Bacilli, order Lactobacillales, are preferable, and those belonging to Aerococcaceae, Camobacteriaceae, Enterococcaceae, Streptococcaceae, Lactobacillaceae, Leuconostocaceae, and Streptococcaceae are more preferable.

Examples of lactic acid bacteria belonging to Lactobacillaceae include bacteria of the genus Lactobacillus, such as Lactobacillus gasseri, L. acidophilus, L. helveticus, L. paracasei, L. casei, L. rhamunosus, L. delbrueckii, L. delbrueckii subsp. bulgaricus, and L. plantarum.

Examples of lactic acid bacteria belonging to Enterococcaceae include bacteria of the genus Enterococcus, such as Enterococcus faecalis and E. faecium.

Examples of lactic acid bacteria belonging to Streptococcaceae include bacteria of the genus Lactococcus, such as Lactococcus lactis and L. lactis subsp. cremoris, and bacteria of the genus Streptococcus, such as Streptococcus thermophilus.

Examples of lactic acid bacteria belonging to Leuconostocaceae include bacteria of the genus Leuconostoc, such as Leuconostoc mesenteroides and L. mesenteroides subsp. cremoris.

As the lactic acid bacteria, at least one selected from the group consisting of the above bacterial species is preferable. In addition, as the lactic acid bacteria, it is also possible to use bacterial strains of an identified genus or epithet or newly discovered bacteria strains.

Lactic acid bacteria can be used in the form of live cells or killed cells. The mode of the lactic acid bacteria used may also be frozen, freeze-dried, or spray-dried. Further, the lactic acid bacteria may contain only bacterial cells of lactic acid bacteria, or may also contain, in addition to bacterial cells of lactic acid bacteria, components other than bacterial cells (e.g., cryoprotectants, freeze-drying protectants, spray drying protectants, etc.). In addition, the lactic acid bacteria may also be in the state of being dispersed in a triturate. The triturate used may be a starch such as corn starch, potato starch, or tapioca starch, a starch decomposed product, dextrin, maltodextrin, or the like.

### (Lactobacillus gasseri)

Lactobacillus gasseri is not particularly limited as long as beneficial effects are exerted on the host. Specifically, Lactobacillus gasseri is not particularly limited as long as beneficial effects are exerted on the host by the bacteria alone or in combination with other active ingredients.

Specific examples of Lactobacillus gasseri include NITE BP-01669, ATCC 33323, DSM 20243, JCM 1131, SBT 2055, and OLL 2716. As Lactobacillus gasseri, NITE BP-01669 is particularly preferable. A single strain of Lactobacillus gasseri may be used alone, and it is also possible to use a combination of two or more strains.

### (Lactobacillus acidophilus)

Lactobacillus acidophilus is not particularly limited as long as beneficial effects are exerted on the host. Specifically, Lactobacillus acidophilus is not particularly limited as long as beneficial effects are exerted on the host by the bacteria alone or in combination with other active ingredients.

Specific examples of Lactobacillus acidophilus include NITE BP-01695, ATCC 4356, DSM 20079, JCM 1132, YIT 0168, and YIT 0154. As Lactobacillus acidophilus, NITE BP-01695 is particularly preferable. A single strain of Lactobacillus acidophilus may be used alone, and it is also possible to use a combination of two or more strains.

### (Lactobacillus helveticus)

Lactobacillus helveticus is not particularly limited as long as beneficial effects are exerted on the host. Specifically, Lactobacillus helveticus is not particularly limited as long as beneficial effects are exerted on the host by the bacteria alone or in combination with other active ingredients.

Specific examples of Lactobacillus helveticus include NITE BP-01671, ATCC 15009, DSM 20075, JCM 1120, and SBT 2171. As Lactobacillus helveticus, NITE BP-01671 is particularly preferable. A single strain of Lactobacillus helveticus may be used alone, and it is also possible to use a combination of two or more strains.

### (Lactobacillus paracasei)

Lactobacillus paracasei is not particularly limited as long as beneficial effects are exerted on the host. Specifically, Lactobacillus paracasei is not particularly limited as long as beneficial effects are exerted on the host by the bacteria alone or in combination with other active ingredients.

Specific examples of Lactobacillus paracasei include NITE BP-01633, ATCC 25302, DSM 5622, JCM 8130, ATCC 25599, DSM 20258, and JCM 1171. As Lactobacillus paracasei, NITE BP-01633 is particularly preferable. A single strain of Lactobacillus paracasei may be used alone, and it is also possible to use a combination of two or more strains.

### (Culturing Method for Lactic Acid Bacteria)

Bacterial cells of lactic acid bacteria can be easily acquired by culturing lactic acid bacteria. The culturing method is not particularly limited as long as lactic acid bacteria can grow. As the culturing method, for example, a method commonly used for culturing lactic acid bacteria can be used directly or after suitable modification. The culture temperature is preferably 25 to 50°C, and more preferably 35 to 42°C. The culture is preferably performed under anaerobic conditions. For example, the culture can be performed while passing a non-oxidizing gas, such as carbon dioxide. In addition, culture under microaerophilic conditions, such as liquid stationary culture, is also possible. The culture may be performed, for example, until lactic acid bacteria grow to the desired degree.

The medium used for culture is not particularly limited as long as lactic acid bacteria can grow. As the medium, for example, a medium commonly used for culturing lactic acid bacteria can be used directly or after suitable modification. That is, as carbon sources, for example, saccharides such as galactose, glucose, fructose, mannose, cellobiose, maltose, lactose, sucrose, trehalose, starches, starch hydrolysates, and blackstrap molasses can be used according to the utilization. In addition, culture in a medium containing a milk protein such as casein or whey, or a decomposition product thereof, is also possible. As nitrogen sources, for example, ammonia, as well as ammonium salts and nitrate salts, such as ammonium sulfate, ammonium chloride, and ammonium nitrate, can be used. In addition, as inorganic salts, for example, sodium chloride, potassium chloride, potassium phosphate, magnesium sulfate, calcium chloride, calcium nitrate, manganese chloride, ferrous sulfate, and the like can be used. In addition, organic components such as peptone, soybean flour, defatted soybean cake, meat extracts, and yeast extracts may also be used. In addition, as a prepared medium, an MRS medium (de Man, Rogosa, and Sharpe medium) can be used, for example.

### <<Bacteria of the Genus Bifidobacterium>>

Bifidobacterium is the genus name for a group of bacteria belonging to the phylum Actinobacteria, class Actinobacteria, order Bifidobacteriales, in the domain Bacteria.

Examples of bacteria of the genus Bifidobacterium include B. longum subsp. infantis, B. breve, B. longum subsp. longum, B. longum subsp. suis, B. animalis subsp. lactis, B. animalis subsp. animalis, B. bifidum, B. adolescentis, B. angulatum, B. dentium, B. pseudocatenulatum, B. pseudolongum, and B. thermophilum.

As the bacteria of the genus Bifidobacterium, it is preferable to use at least one selected from the group consisting of B. longum subsp. infantis, B. breve, B. longum subsp. longum, B. longum subsp. suis, B. animalis subsp. lactis, and B. bifidum, and it is more preferable to use at least one selected from the group consisting of B. longum subsp. infantis, B. breve, B. longum subsp. longum, and B. animalis subsp. lactis.

A single species of bacteria of the genus Bifidobacterium may be used alone, or it is also possible to use a combination of two or more species.

As a combination in the case of using two or more species of bacteria of the genus Bifidobacterium together, a combination of at least one subspecies of Bifidobacterium longum with Bifidobacterium breve is preferable.

As the bacteria of the genus Bifidobacterium, it is also possible to use bacterial strains of an identified epithet or newly discovered bacteria strains.

Bacteria of the genus Bifidobacterium can be used in the form of live cells or killed cells. The mode of the bacteria of the genus Bifidobacterium used may also be frozen, freeze-dried, or spray-dried. Further, the bacteria of the genus Bifidobacterium may contain only bacterial cells of bacteria of the genus Bifidobacterium, or may also contain, in addition to bacterial cells of bacteria of the genus Bifidobacterium, components other than bacterial cells (e.g., cryoprotectants, freeze-drying protectants, spray-drying protectants, etc.). In addition, the bacteria of the genus Bifidobacterium may also be in the state of being dispersed in a triturate. The triturate used may be a starch such as corn starch, potato starch, or tapioca starch, a starch decomposed product, dextrin, maltodextrin, or the like.

### (Bifidobacterium longumlongum subspecies infantis)

Bifidobacterium longum subspecies infantis is not particularly limited as long as beneficial effects are exerted on the host. Specifically, Bifidobacterium longum subspecies infantis is not particularly limited as long as beneficial effects are exerted on the host by the bacteria alone or in combination with other active ingredients.

Specific examples of Bifidobacterium longum subspecies infantis include NITE BP-02623, ATCC 15697, ATCC 15702, DSM 20088, and JCM 1222. As Bifidobacterium longum subspecies infantis, NITE BP-02623 is particularly preferable. A single strain of Bifidobacterium longum subspecies infantis may be used alone, and it is also possible to use a combination of two or more strains.

### (Bifidobacterium breve)

Bifidobacterium breve is not particularly limited as long as beneficial effects are exerted on the host. Specifically, Bifidobacterium breve is not particularly limited as long as beneficial effects are exerted on the host by the bacteria alone or in combination with other active ingredients.

Specific examples of Bifidobacterium breve include NITE BP-02622, FERM BP-11175, ATCC 15700, ATCC 15698, DSM 20213, DSM 24706, DSM 13692, DSM 24732, DSM 24736, DSM 16604, JCM 1192, NCC 2705, NCC490, YIT 4010, YIT 4064, SBT 2928, UCC 2003, BBG-001, C 50, R 0070, and BG 7. As Bifidobacterium breve, NITE BP-02622 is particularly preferable. A single strain of Bifidobacterium breve may be used alone, and it is also possible to use a combination of two or more strains.

### (Bifidobacterium longum subspecies longum)

Bifidobacterium longum subspecies longum is not particularly limited as long as beneficial effects are exerted on the host. Specifically, Bifidobacterium longum subspecies longum is not particularly limited as long as beneficial effects are exerted on the host by the bacteria alone or in combination with other active ingredients.

Specific examples of Bifidobacterium longum subspecies longum include NITE BP-02621, ATCC 15707, ATCC 25962, DSM 20219, and JCM 1217. As Bifidobacterium longum subspecies longum, NITE BP-02621 is particularly preferable. A single strain of Bifidobacterium longum subspecies longum may be used alone, and it is also possible to use a combination of two or more strains.

### (Bifidobacterium longum subspecies suis)

Bifidobacterium longum subspecies suis is not particularly limited as long as beneficial effects are exerted on the host. Specifically, Bifidobacterium longum subspecies suis is not particularly limited as long as beneficial effects are exerted on the host by the bacteria alone or in combination with other active ingredients.

Specific examples of Bifidobacterium longum subspecies suis include ATCC 27533, ATCC 27532, DSM 20211, and JCM 1269. As Bifidobacterium longum subspecies suis, ATCC 27533 is particularly preferable. A single strain of Bifidobacterium longum subspecies suis may be used alone, and it is also possible to use a combination of two or more strains.

### (Bifidobacterium animalis subspecies lactis)

Bifidobacterium animalis subspecies lactis is not particularly limited as long as beneficial effects are exerted on the host. Specifically, Bifidobacterium animalis subspecies lactis is not particularly limited as long as beneficial effects are exerted on the host by the bacteria alone or in combination with other active ingredients.

Specific examples of Bifidobacterium animalis subspecies lactis include DSM 15954 and FERM P-21998. As Bifidobacterium animalis subspecies lactis, DSM 15954 is particularly preferable. A single strain of Bifidobacterium animalis subspecies lactis may be used alone, and it is also possible to use a combination of two or more strains.

Incidentally, in place of the strains identified by the strain numbers mentioned as examples, strains that are substantially the same as the strains stored in a culture collection under such strain numbers may also be used. For example, in the case of Bifidobacterium longum subspecies longum, ATCC 15707 may be replaced with DSM 20219 or JCM 1217. As an indicator of whether strains are substantially the same, the 16S rRNA gene base sequence identity can be used, for example. When strains are substantially the same, the 16S rRNA gene base sequence identity is preferably 99.86% or more, more preferably 99.93% or more, and still more preferably 100%. When strains are substantially the same, it is particularly preferable that they have 100% 16S rRNA gene base sequence identity, and also are the same in terms of microbiological properties such as utilization performance.

In addition, in place of the strains identified by the strain numbers mentioned as examples, derivatives of the strains may also be used. Examples of derivatives include strains artificially bred from stocks and strains spontaneously generated from stocks. Examples of breeding methods include modification by genetic engineering and modification by mutation treatment. The mutation treatment may be, for example, X-ray irradiation, UV irradiation, or treatment with a mutation agent such as N-methyl-N'-nitro-N-nitrosoguanidine (MNNG), ethyl methanesulfonate (EMS), or methyl methanesulfonate (MMS). Examples of strains spontaneously generated from stocks include mutants spontaneously generated upon use of the stocks. The derivative may be constructed by the modification of one strain or may also be constructed by the modification of two or more strains.

The bacteria of the genus Bifidobacterium used may be a commercially available product or may also be suitably produced and acquired. Examples of commercially available products include Bifidobacterium longum subspecies longum NITE BP-02621, Bifidobacterium breve NITE BP-02622, Bifidobacterium longum subspecies infantis NITE BP-02623, and Bifidobacterium animalis subspecies lactis BB-12 (DSM 15954).

### (Culturing Method for Bacteria of the Genus Bifidobacterium)

Bacterial cells of bacteria of the genus Bifidobacterium can be easily acquired by culturing bacteria of the genus Bifidobacterium. The culturing method is not particularly limited as long as bacteria of the genus Bifidobacterium can grow. As the culturing method, for example, a method commonly used for culturing bacteria of the genus Bifidobacterium can be used directly or after suitable modification. The culture temperature is preferably 25 to 50°C, and more preferably 35 to 42°C. The culture is preferably performed under anaerobic conditions. For example, the culture can be performed while passing a non-oxidizing gas, such as carbon dioxide. In addition, culture under microaerophilic conditions, such as liquid stationary culture, is also possible. The culture may be performed, for example, until bacteria of the genus Bifidobacterium grow to the desired degree.

The medium used for culture is not particularly limited as long as bacteria of the genus Bifidobacterium can grow. As the medium, for example, a medium commonly used for culturing bacteria of the genus Bifidobacterium can be used directly or after suitable modification. That is, as carbon sources, for example, saccharides such as galactose, glucose, fructose, mannose, cellobiose, maltose, lactose, sucrose, trehalose, starches, starch hydrolysates, and blackstrap molasses can be used according to the utilization. In addition, culture in a medium containing a milk protein such as casein or whey, or a decomposition product thereof, is also possible. As nitrogen sources, for example, ammonia, as well as ammonium salts and nitrate salts, such as ammonium sulfate, ammonium chloride, and ammonium nitrate, can be used. In addition, as inorganic salts, for example, sodium chloride, potassium chloride, potassium phosphate, magnesium sulfate, calcium chloride, calcium nitrate, manganese chloride, ferrous sulfate, and the like can be used. In addition, organic components such as peptone, soybean flour, defatted soybean cake, meat extracts, and yeast extracts may also be used. In addition, as a prepared medium, an MRS medium (de Man, Rogosa, and Sharpe medium) can be used, for example.

### <<Acronyms of Culture Collections>>

The acronyms of culture collections are as follows.
NITE: NITE Patent Microorganisms Depositary (NPMD), National Institute of Technology and Evaluation
FERM: NITE Patent Microorganisms Depositary (NPMD), National Institute of Technology and Evaluation
ATCC: American Type Culture Collection
DSM: Deutsche Sammlung von Mikroorganismen und Zellkulturen GmbH (DSMZ)
JCM: Japan Collection of Microorganisms, Riken BRC
NCC: Nestle S.A.
YIT: Yakult Honsha Co., Ltd.
SBT: MEGMILK Snow Brand Co., Ltd.
OLL: Meiji Co., Ltd.
R: LALLEMAND Inc.

### <<Probiotics>>

The bacterial powder may be live cells or killed cells. In the case where the bacterial powder is live cells, such a bacterial powder can be expected to function as a probiotic.

Incidentally, "probiotics" is a term proposed as opposed to "antibiotics", and is derived from the term "probiosis", which means to live together. The definition of probiotics currently accepted widely is "living microorganisms that improve the balance of intestinal flora and thereby beneficially affect the host's health".

### <<Content of Bacterial powder>>

The content of the bacterial powder in the composition of the invention is preferably 0.5 to 10 mass%, more preferably 1 to 5 mass%, based on the total mass of the composition.

When the content of the bacterial powder is 0.5 mass% or more based on the total mass of the composition, the advantageous effect of the presence of the bacterial powder in the composition of the invention can be more exerted.

When the content of the bacterial powder is 10 mass% or less based on the total mass of the composition, the advantageous effect of the presence of the bacterial powder in the composition of the invention and the cost are more balanced.

### <Fatty Oil>

As the fatty oil in the invention, an oil that is liquid in the course of distributing the composition of the invention, for example, in at least a part of a range of 0 to 40°C, is preferable, and an oil that is liquid within the entire range of 0 to 40°C is more preferable.

As the fatty oil, an edible oil is preferable.

Examples of edible oils include hazelnut oil, olive oil, primula oil, pumpkin oil, rice bran oil, soybean oil, corn oil, sunflower oil, rapeseed oil, safflower oil, coconut oil (including cohune oil, saw palmetto oil, etc.), palm oil, palm kernel oil, medium chain triglyceride (MCT), docosahexaenoic acid (DHA), eicosapentaenoic acid (EPA), linseed oil, perilla oil, rice germ oil, wheat germ oil, coconut oil, cottonseed oil, peanut oil, sesame oil, almond oil, cashew oil, macadamia nut oil, mongongo oil, pecan oil, pine nut oil, pistachio oil, walnut oil, bottle gourd oil, buffalo gourd oil, pumpkin seed oil, watermelon seed oil, acai berry extract, blackcurrant seed oil, borage seed oil, evening primrose oil, amaranth oil,
apricot oil, apple seed oil, argan oil, artichoke oil, avocado oil, babassu oil, moringa oil, cape chestnut oil, carob oil, coriander oil, dika oil, false flax oil, grape seed oil, hemp oil, kapok seed oil, lallemantia oil, marula oil, meadowfoam seed oil, mustard oil, okra oil (hibiscus oil), papaya oil, poppy seed oil, prune kernel oil, quinoa oil, niger seed oil, tea seed oil (camellia seed oil), thistle oil, tomato seed oil, krill oil, and borage oil.

It is preferable that the fatty oil is at least one selected from the group consisting of olive oil, rice bran oil, soybean oil, corn oil, sunflower oil, safflower oil, and medium chain triglyceride (MCT), more preferably at least one selected from the group consisting of medium chain triglyceride (MCT), corn oil, and sunflower oil, and particularly preferably medium chain triglyceride (MCT).

The fatty oil is contained in an amount of 90 mass% or more, more preferably 92 mass% or more, and still more preferably 95 mass% or more, based on the total mass of the composition.

### <Microscopic Powder and Surfactant>

In the composition of the invention, a microscopic powder and a surfactant act as anti-caking agents.

An anti-caking agent acts to prevent or dissolve caking.

### <<Microscopic Powder>>

The microscopic powder is a microscopic-size powder of an organic substance or an inorganic substance, and is preferably a microscopic-size powder of an organic substance.

As a microscopic-size powder of an organic substance, for example, microcrystalline cellulose can be mentioned. Microcrystalline cellulose is high-purity cellulose obtained by hydrolyzing and purifying pulp with an acid.

The average particle size of microcrystalline cellulose particles is, as D50, preferably 1 to 200 µm, and more preferably 5 to 100 µm.

The average particle size (D50) of microscopic particles is a 50% volume particle size calculated from the volume distribution determined by a laser diffraction/scattering method.

As a microscopic-size powder of an inorganic substance, for example, tricalcium phosphate and fine silicon dioxide can be mentioned. Tricalcium phosphate is a salt of phosphoric acid and calcium represented by chemical formula Ca₃(PO₄)₂. Tricalcium phosphate has three types of polymorphs. In the composition of the invention, β-tricalcium phosphate (β-TCP), which is a low-temperature polymorph, is observed. Fine silicon dioxide is microscopic particles of silica. As a microscopic-size powder of the invention tricalcium phosphate or microcrystalline cellulose is used.

The average particle size of tricalcium phosphate particles is, as D50, preferably 1 to 200 µm, and more preferably 1 to 50 µm.

According to the invention, the microscopic powder contains at least one selected from the group consisting of microcrystalline cellulose and tricalcium phosphate.

In the composition of the invention, a microscopic powder and a surfactant are suitable for use as anti-caking agents.

### <<Surfactant>>

The surfactant is at least one selected from the group consisting of an anionic surfactant and a nonionic surfactant having an HLB value of less than 7.

### (Anionic Surfactant)

Examples of anionic surfactants include carboxylic acid anionic surfactants, sodium linear alkylbenzene sulfonates, sulfonic acid anionic surfactants, sulfate anionic surfactants, and phosphate anionic surfactants.

The anionic surfactant is preferably at least one selected from the group consisting of salts of C₁₂₋₁₈ fatty acids, more preferably at least one selected from the group consisting of metal stearates, still more preferably an alkaline earth metal salt of stearic acid, and particularly preferably calcium stearate or magnesium stearate.

A single kind of anionic surfactant may be used alone, or it is also possible to use a combination of two or more kinds.

### (Nonionic Surfactant Having HLB Value of Less Than 7)

The nonionic surfactant having an HLB value of less than 7 is preferably a fatty acid ester having an HLB value of less than 7, more preferably at least one selected from the group consisting of sucrose fatty acid esters having an HLB value of less than 7 and glycerin fatty acid esters having an HLB value of less than 7, and still more preferably at least one selected from the group consisting of sucrose fatty acid esters having an HLB value of less than 7.

A single kind of nonionic surfactant having an HLB value of less than 7 may be used alone, or it is also possible to use a combination of two or more kinds.

### <<Contents of Microscopic Powder and Surfactant>>

The contents of the microscopic powder and the surfactant in the composition of the invention are preferably such that
the total content of the microscopic powder and the surfactant is 0.01 to 10 mass%, more preferably 0.1 to 5 mass%, and still more preferably 0.5 to 2 mass%, based on the total mass of the composition.

### <Additive>

The composition of the invention may further contain additives in addition to the components described above.

Examples of additives include antioxidants, excipients, binders, disintegrators, lubricants, stabilizers, flavoring agent, and diluents.

As an antioxidant, vitamin E is preferable. Vitamin E is a fat-soluble vitamin and easy to dissolve in the fatty oil in the composition.

### <Intended Use of Composition>

The composition of the invention may be a supplement composition, a beverage composition, a food composition, a pharmaceutical composition, or an animal feed composition, for example, but is preferably used as a supplement composition.

In the case where the composition of the invention is used as a supplement composition, the composition of the invention may be directly ingested. Alternatively, the composition of the invention may also be added to a supplement, a beverage, a food, a pharmaceutical, or an animal feed and ingested.

In the case where the composition of the invention is added to a supplement, a beverage, a food, a pharmaceutical, or an animal feed, it is preferable that some drops of the composition of the invention are added to a beverage, a food, a pharmaceutical, or an animal feed, and thus utilized.

### [Production Method]

The composition of the invention can be produced by mixing a bacterial powder, a fatty oil, and at least one selected from the group consisting of a microscopic powder and a surfactant in an arbitrary order.

The composition of the invention can also be produced by mixing a suspension containing a bacterial powder and a fatty oil with at least one selected from the group consisting of a microscopic powder and a surfactant.

The mixing method is not particularly limited. For example, the bacterial powder, fatty oil, and at least one selected from the group consisting of a microscopic powder and a surfactant can be mixed by stirring.

In the case where the composition contains additives, the order of mixing them is not particularly limited. The additives may be contained in a suspension containing a bacterial powder and a fatty oil, and it is also possible to add the additives at the time of mixing at least one selected from the group consisting of a microscopic powder and a surfactant.

### [Use]

The microscopic powder and surfactant described above are used as anti-caking agents.

### [Examples]

Hereinafter, the invention will be described in further detail with reference to examples. However, the invention is not limited to the following examples. Unless the gist of the invention is changed, various modifications are possible.

### [Preparation of Bacterial powder]

### <Bacterial powder 1>

Bifidobacterium infantis NITE BP-02623 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial cell mass was physically ground to give a freeze-dried powder. The obtained freeze-dried powder and tapioca starch were triturated in a ratio of 1:3 (w/w), thereby giving a Bifidobacterium bacteria bacterial powder.

### <Bacterial powder 2>

Bifidobacterium breve NITE BP-02622 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 120 hours, and the freeze-dried bacterial cell mass was physically ground to give a freeze-dried powder. The obtained freeze-dried powder and tapioca starch were triturated in a ratio of 1:3 (w/w), thereby giving a Bifidobacterium bacteria bacterial powder.

### <Bacterial powder 3>

Bifidobacterium longum subsp. longum NITE BP-02621 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial cell mass was physically ground to give a freeze-dried powder. The obtained freeze-dried powder and corn starch were triturated in a ratio of 1:3 (w/w), thereby giving a Bifidobacterium bacteria bacterial powder.

### <Bacterial powder 4>

Bifidobacterium longum subsp. longum NITE BP-02621 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial cell mass was physically ground to give a freeze-dried powder. The obtained freeze-dried powder and potato starch were triturated in a ratio of 1:3 (w/w), thereby giving a Bifidobacterium bacteria bacterial powder.

### <Bacterial powder 5>

Lactobacillus gasseri NITE BP-01669 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial cell mass was physically ground to give a freeze-dried powder. The obtained freeze-dried powder and corn starch were triturated in a ratio of 1:3 (w/w), thereby giving a lactic acid bacterial powder.

### <Bacterial powder 6>

Lactobacillus acidophilus NITE BP-01695 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial cell mass was physically ground to give a freeze-dried powder. The obtained freeze-dried powder and corn starch were triturated in a ratio of 1:3 (w/w), thereby giving a lactic acid bacterial powder.

### <Bacterial powder 7>

Lactobacillus paracasei NITE BP-01633 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial cell mass was physically ground to give a freeze-dried powder. The obtained freeze-dried powder and maltodextrin were triturated in a ratio of 1:3 (w/w), thereby giving a lactic acid bacterial powder.

### <Bacterial powder 8>

Lactobacillus helveticus NITE BP-01671 was inoculated into a medium containing a protein, an amino acid, and a sugar source, cultured at 32 to 41°C for 5 to 24 hours, and then centrifuged to harvest bacterial cells (wet bacterial cells) from the culture solution. A concentrated bacterial solution and a starch decomposed product were mixed in a ratio of 1:4 (on a solids basis, w/w) and then spray-dried using a spray dryer, thereby giving a lactic acid bacterial powder.

### <Bacterial powder 9>

Bifidobacterium longum subsp. infantis NITE BP-02623 was inoculated into a medium containing a protein, an amino acid, and a sugar source, cultured at 32 to 41°C for 5 to 24 hours, and then centrifuged to harvest bacterial cells (wet bacterial cells) from the culture solution. Using a freeze-dryer (manufactured by Kyowa Vacuum Engineering Co., Ltd.), freeze-drying was performed for 18 to 96 hours, and the freeze-dried bacterial mass was physically ground to give a freeze-dried powder.

### [Dispersibility Evaluation Method]

10 mL of the prepared composition was placed in a test tube made of glass, followed by sealing with a rubber plug. The test tube containing the composition was allowed to stand in an incubator set at 5°C for 30 days. The composition after standing was mixed by inversion 20 times at a speed of approximately once per second, and then the bottom surface of the test tube was observed.

According to the following criteria, dispersibility was evaluated on a five-grade scale from A to E.
A: No bacterial powder or carrier remained on the bottom surface.
B: The bacterial powder or carrier more remained on the bottom surface than in A.
C: The bacterial powder or carrier more remained on the bottom surface than in B.
D: The bacterial powder or carrier more remained on the bottom surface than in C.
E: Suspension was difficult.

### [Reference Example]

2.5 mass% of tapioca starch (Reference Example 1) or maltodextrin (Reference Example 2) was mixed with 97.5 mass% of an MCT oil (S9013) to prepare 100 mass% of a composition.

Using the prepared compositions, dispersibility was evaluated according to the evaluation method described above. The evaluation results are shown in Table 1.

**[Table 1]**

| | Reference Example 1 | Reference Example 2 |
|---|---|---|
| Evaluation | C | B |

In Reference Example 1 and Reference Example 2 where no bacterial powder was blended, the dispersibility of the carrier was excellent. This suggested that caking of a bacterial powder in oil drops was caused by bacterial cells themselves contained in the bacterial powder.

### [Examples and Comparative Examples]

### <Example to Example A22>

Example A1 and Examples A5 to A7 are comparative examples.

In the ratio shown in Table 2 or Table 3 (unit: mass%), a bacterial powder, a microscopic powder or a surfactant, and a fatty oil were mixed and formed into a homogeneous dispersion liquid using a magnetic stirrer. Of the surfactants, B-100D was added to a fatty oil, dissolved in a hot bath at 90°C, and returned to room temperature, and then a bacterial powder was added and uniformly mixed.

Using the prepared compositions, dispersibility was evaluated according to the evaluation method described above. The evaluation results are shown in Table 2 and Table 3.

**[Table 2]**

| | | A1 | A2 | A3 | A4 | A5 | A6 | A7 | A8 | A9 | A10 |
|---|---|---|---|---|---|---|---|---|---|---|---|
| Bacterial powder | Bacterial powder 1 | 2.5 | 2.5 | 2.5 | 2.5 | 2.5 | 2.5 | 2.5 | 2.5 | 2.5 | 2.5 |
| Microscopic Powder or Surfactant | Microcrystalline cellulose | | 0.5 | 1.0 | 2.0 | | | | | | |
| | Fine silicon dioxide | | | | | 0.5 | 1.0 | 2.0 | | | |
| | Ca stearate | | | | | | | | 0.5 | 1.0 | 2.0 |
| | B-100D | | | | | | | | | | |
| | S-170 | | | | | | | | | | |
| | S-770 | | | | | | | | | | |
| | S-1570 | | | | | | | | | | |
| Fatty oil | S9013 | 97.5 | 97.0 | 96.5 | 95.5 | 97.0 | 96.5 | 95.5 | 97.0 | 96.5 | 95.5 |
| Evaluation | Dispersibility | E | C | C | C | A | A | A | B | B | B |

**[Table 3]**

| | | A11 | A12 | A13 | A14 | A15 | A16 | A17 | A18 | A19 | A20 | A21 | A22 |
|---|---|---|---|---|---|---|---|---|---|---|---|---|---|
| Bacterial powder | Bacterial powder 1 | 2.5 | 2.5 | 2.5 | 2.5 | 2.5 | 2.5 | 2.5 | 2.5 | 2.5 | 2.5 | 2.5 | 2.5 |
| Microscopic Powder or Surfactant | Microcrystalline cellulose | | | | | | | | | | | | |
| | Fine silicon dioxide | | | | | | | | | | | | |
| | Ca stearate | | | | | | | | | | | | |
| | B-100D | 0.5 | 1.0 | 2.0 | | | | | | | | | |
| | S-170 | | | | 0.5 | 1.0 | 2.0 | | | | | | |
| | S-770 | | | | | | | 0.5 | 1.0 | 2.0 | | | |
| | S-1570 | | | | | | | | | | 0.5 | 1.0 | 2.0 |
| Fatty oil | S9013 | 97.0 | 96.5 | 95.5 | 97.0 | 96.5 | 95.5 | 97.0 | 96.5 | 95.5 | 97.0 | 96.5 | 95.5 |
| Evaluation | Dispersibility | A | A | A | C | C | C | D | D | D | D | D | D |

In Example A2 to Example A22 where a microscopic powder or a surfactant was blended, the dispersibility of the bacterial powder was excellent as compared with Example A1 where none of them was blended.

In Example A8 to Example A10 where an anionic surfactant (Ca stearate) was blended, the dispersibility ratings were all B, that is, the dispersibility of the bacterial powder was excellent as compared with Example A1.

In Example A11 to Example A16 where a nonionic surfactant having an HLB value of less than 7 (B-100D, S-170) was blended, the dispersibility of the bacterial powder was excellent as compared with Example A17 to Example A22 where a nonionic surfactant having a HLB value of 7 or more (S-770, S-1570) was blended.

### <Example B1 to Example B9>

Example B1 is a comparative example, and Example B2 to Example B9 are inventive.

In the ratio shown in Table 4 (unit: mass%), a bacterial powder, a microscopic powder or a surfactant, and a fatty oil were mixed and formed into a homogeneous dispersion liquid using a magnetic stirrer. Of the surfactants, L-195 and POS-135 were added to a fatty oil, dissolved in a hot bath at 90°C, and returned to room temperature, and then a bacterial powder was added and uniformly mixed.

Using the prepared compositions, dispersibility was evaluated according to the evaluation method described above. The evaluation results are shown in Table 4.

**[Table 4]**

| | | B1 | B2 | B3 | B4 | B5 | B6 | B7 | B8 | B9 |
|---|---|---|---|---|---|---|---|---|---|---|
| Bacterial powder | Bacterial powder 1 | 2.5 | 2.5 | 2.5 | 2.5 | 2.5 | 2.5 | 2.5 | 2.5 | 2.5 |
| Microscopic Powder or Surfactant | Tri-Ca phosphate | | 1.0 | | | | | | | |
| | Mg stearate | | | 1.0 | | | | | | |
| | P-170 | | | | 1.0 | | | | | |
| | O-170 | | | | | 1.0 | | | | |
| | L-195 | | | | | | 1.0 | | | |
| | B-370F | | | | | | | 1.0 | | |
| | ER-190 | | | | | | | | 1.0 | |
| | PCS-135 | | | | | | | | | 1.0 |
| Fatty oil | S9013 | 97.5 | 96.5 | 96.5 | 96.5 | 96.5 | 96.5 | 96.5 | 96.5 | 96.5 |
| Evaluation | Dispersibility | E | A | A | B | B | C | A | C | A |

In Example B2 to Example B9 where a microscopic powder or a surfactant was blended, the dispersibility of the bacterial powder was excellent as compared with Example B1 where none of them was blended.

In Example B2 where tri-Ca phosphate was blended as a microscopic powder, Example B3 where Mg stearate was blended as an anionic surfactant, Example B7 where B-370F (HLB value: 3) was blended as a nonionic surfactant, and Example B9 where POS-135 (HLB value: 1) was blended as a nonionic surfactant, the dispersibility ratings were A, that is, the dispersibility of the bacterial powder was particularly excellent.

### <Example C1 to Example C11>

Example C1 is a comparative example, and Example C2 to Example C11 are inventive.

In the ratio shown in Table 5 (unit: mass%), a bacterial powder, a microscopic powder or a surfactant, and a fatty oil were mixed and formed into a homogeneous dispersion liquid using a magnetic stirrer. Of the surfactants, B-100D was added to a fatty oil, dissolved in a hot bath at 90°C, and returned to room temperature, and then a bacterial powder was added and uniformly mixed.

Using the prepared compositions, dispersibility was evaluated according to the evaluation method described above. The evaluation results are shown in Table 5.

**[Table 5]**

| | | C1 | C2 | C3 | C4 | C5 | C6 | C7 | C8 | C9 | C10 | C11 |
|---|---|---|---|---|---|---|---|---|---|---|---|---|
| Bacterial powder | Bacterial powder 2 | 2.5 | 2.5 | 2.5 | 2.5 | 2.5 | 2.5 | 2.5 | 2.5 | 2.5 | 2.5 | 2.5 |
| Microscopic Powder or Surfactant | Microcrystalline cellulose | | 0.5 | 1.0 | 2.0 | | | | | | | |
| | Tri-Ca phosphate | | | | | 1.0 | | | | | | |
| | Ca stearate | | | | | | 1.0 | | | | | |
| | Mg stearate | | | | | | | 1.0 | | | | |
| | B-100D | | | | | | | | 1.0 | | | |
| | S-170 | | | | | | | | | 1.0 | | |
| | P-170 | | | | | | | | | | 1.0 | |
| | B-370F | | | | | | | | | | | 1.0 |
| Fatty oil | S9013 | 97.5 | 97.0 | 96.5 | 95.5 | 96.5 | 96.5 | 96.5 | 96.5 | 96.5 | 96.5 | 96.5 |
| | Evaluation | E | D | C | C | A | B | B | A | C | B | A |

In Example C2 to Example C11 where a microscopic powder or a surfactant was blended, the dispersibility of the bacterial powder was excellent as compared with Example C1 where none of them was blended.

In Example C5 where tri-Ca phosphate was blended as a microscopic powder, Example C8 where B-100D (HLB value: 3) was blended as a nonionic surfactant, and Example C11 where B-370F (HLB value: 3) was blended as a nonionic surfactant, the dispersibility ratings were A, that is, the dispersibility of the bacterial powder was particularly excellent.

### <Example D1 to Example D12>

Example D1, Example D3, Example D5, Example D7, Example D9, and Example D11 are comparative examples, and Example D2, Example D4, Example D6, Example D8, Example D10, and Example D12 are inventive.

In the ratio shown in Table 6 (unit: mass%), a bacterial powder, a microscopic powder or a surfactant, and a fatty oil were mixed and formed into a homogeneous dispersion liquid using a magnetic stirrer.

Using the prepared compositions, dispersibility was evaluated according to the evaluation method described above. The evaluation results are shown in Table 6.

**[Table 6]**

| | | D1 | D2 | D3 | D4 | D5 | D6 | D7 | D8 | D9 | D10 | D11 | D12 |
|---|---|---|---|---|---|---|---|---|---|---|---|---|---|
| Bacterial powder | Bacterial powder 3 | 2.5 | 2.5 | | | | | | | | | | |
| | Bacterial powder 4 | | | 2.5 | 2.5 | | | | | | | | |
| | Bacterial powder 5 | | | | | 2.5 | 2.5 | | | | | | |
| | Bacterial powder 6 | | | | | | | 2.5 | 2.5 | | | | |
| | Bacterial powder 7 | | | | | | | | | 2.5 | 2.5 | | |
| | Bacterial powder 8 | | | | | | | | | | | 2.5 | 2.5 |
| Microscopic Powder or Surfactant | Ca stearate | | 1.0 | | 1.0 | | 1.0 | | 1.0 | | 1.0 | | 1.0 |
| Fatty oil | S9013 | 97.5 | 96.5 | 97.5 | 96.5 | 97.5 | 96.5 | 97.5 | 96.5 | 97.5 | 96.5 | 97.5 | 96.5 |
| Evaluation | Dispersibility | E | A | E | B | E | A | E | B | E | B | E | B |

In Example D2, Example D4, Example D6, Example D8, Example D10, and Example D12 where Ca stearate was blended, the dispersibility ratings were A or B, that is, the dispersibility of the bacterial powder was excellent.

Between the examples using a freeze-dried bacterial powder (Example D2, Example D4, Example D6, Example D8, and Example D10) and the example using a spray-dried bacterial powder (Example D12), no significant difference was observed in dispersibility upon the addition of Ca stearate.

### <Example E1 and Example E2>

Example E1 is a comparative example, and Example E2 is inventive.

In the ratio shown in Table 7 (unit: mass%), a bacterial powder, a microscopic powder or a surfactant, and a fatty oil were mixed and formed into a homogeneous dispersion liquid using a magnetic stirrer.

Using the prepared compositions, dispersibility was evaluated according to the evaluation method described above. The evaluation results are shown in Table 7.

**[Table 7]**

| | | E1 | E2 |
|---|---|---|---|
| Bacterial powder | Bacterial powder 9 | 2.5 | 2.5 |
| Microscopic Powder or Surfactant | Ca stearate | | 1.0 |
| Fatty oil | S9013 | 97.5 | 96.5 |
| Evaluation | Dispersibility | E | B |

In Example E2 where Ca stearate was blended, the dispersibility rating was B, that is, the dispersibility of the bacterial powder was excellent.

Even in the case of using a bacterial powder with no triturate mixed, the addition of Ca stearate resulted in improved dispersibility.

### <Example F 1 and Example F2>

Example F1 is a comparative example, and Example F2 is inventive.

A commercially available supplement composition (Babies' Pro Bio Bifidus M1, sold by Bean Stalk Snow Co., Ltd.) was, directly (Example F1) or after adding 1.0 mass% of Ca stearate (Example F2), used to prepare a composition for evaluation.

The above supplement composition is a composition made from a bifidobacteria bacterial powder (Bifidobacterium animalis subspecies lactis BB-12: DSM 15954), sunflower oil, an antioxidant (vitamin E), and citric acid.

Using the prepared compositions for evaluation, dispersibility was evaluated according to the evaluation method described above. The evaluation results are shown in Table 8.

**[Table 8]**

| | F1 | F2 |
|---|---|---|
| Evaluation | E | C |

Even in the case of a commercially supplement composition, the addition of Ca stearate resulted in improved dispersibility.

The terms in Table 2 to Table 7 have the following meanings.

### (Bacterial powder)

Bacterial powder 1: Bacterial powder 1 prepared as above
Bacterial powder 2: Bacterial powder 2 prepared as above
Bacterial powder 3: Bacterial powder 3 prepared as above
Bacterial powder 4: Bacterial powder 4 prepared as above
Bacterial powder 5: Bacterial powder 5 prepared as above
Bacterial powder 6: Bacterial powder 6 prepared as above
Bacterial powder 7: Bacterial powder 7 prepared as above
Bacterial powder 8: Bacterial powder 8 prepared as above
Bacterial powder 9: Bacterial powder 9 prepared as above

### (Fatty Oil)

S9013: Medium chain triglyceride (MCT Oil S9013, manufactured by Taiyo Yushi Corp.)

### (Microscopic Powder)

Microcrystalline cellulose: CEOLUS FD-F20 (manufactured by Asahi Kasei Corporation)
Tri-Ca phosphate: Tricalcium phosphate (manufactured by Taihei Chemical Industrial Co., Ltd.)
Fine silicon dioxide: SYLOPAGE 720 (manufactured by Fuji Silysia Chemical Ltd.)

### (Surfactant)

Ca stearate: Calcium stearate (manufactured by Taihei Chemical Industrial Co., Ltd.)
Mg stearate: Magnesium stearate (manufactured by San-Ei Gen F.F.I., Inc.)
B-100D: Glycerin fatty acid ester (Ryoto Polyglycerol Ester B-100D, manufactured by Mitsubishi-Chemical Foods Corporation; HLB value: 3)
S-170: Sucrose fatty acid ester (Ryoto Sugar Ester S-170, manufactured by Mitsubishi-Chemical Foods Corporation; HLB value: 1)
P-170: Sucrose fatty acid ester (Ryoto Sugar Ester P-170, manufactured by Mitsubishi-Chemical Foods Corporation; HLB value: 1)
O-170: Sucrose fatty acid ester (Ryoto Sugar Ester 0-170, manufactured by Mitsubishi-Chemical Foods Corporation; HLB value: 1)
L-195: Sucrose fatty acid ester (Ryoto Sugar Ester L-195, manufactured by Mitsubishi-Chemical Foods Corporation; HLB value: 1)
B-370F: Sucrose fatty acid ester (Ryoto Sugar Ester B-370F, manufactured by Mitsubishi-Chemical Foods Corporation; HLB value: 3)
ER-190: Sucrose fatty acid ester (Ryoto Sugar Ester ER-190, manufactured by Mitsubishi-Chemical Foods Corporation; HLB value: 1)
POS-135: Sucrose fatty acid ester (Ryoto Sugar Ester POS-135, manufactured by Mitsubishi-Chemical Foods Corporation; HLB value: 1)
S-770: Sucrose fatty acid ester (Ryoto Sugar Ester S-770, manufactured by Mitsubishi-Chemical Foods Corporation; HLB value: 7)
S-1570: Sucrose fatty acid ester (Ryoto Sugar Ester S-1670, manufactured by Mitsubishi-Chemical Foods Corporation; HLB value: 16)

### [Description of Results]

In the Examples, effectiveness in suppressing the precipitation, deposition, and caking of a bacterial powder was observed.

There was a tendency that those blended with a surfactant showed excellent effects.

Incidentally, from comparison between compositions blended with a bacterial powder and compositions blended only with a carrier, it was confirmed that the compositions blended with a bacterial powder tended to more remain on the bottom surface. Therefore, a composition containing a bacterial powder has a high need for dissolving precipitation/deposition and caking.

### [Industrial Applicability]

The composition of the invention can be directly ingested as a supplement composition or added to a food, and thus utilized

## Claims

1. A composition comprising:
a bacterial powder;
a fatty oil; and
at least one selected from the group consisting of a microscopic powder and a surfactant, wherein the microscopic powder contains at least one selected from the group consisting of microcrystalline cellulose and tricalcium phosphate,
wherein the fatty oil is contained in an amount of 90 mass% or more, based on the total mass of the composition,
and wherein the surfactant contains at least one selected from the group consisting of an anionic surfactant and a nonionic surfactant having an HLB value of less than 7.

2. The composition according to claim 1, being a suspension.

3. The composition according to claim 1 or 2, wherein the bacterial powder contains at least one selected from the group consisting of live cells of lactic acid bacteria, killed cells of lactic acid bacteria, live cells of bacteria of the genus Bifidobacterium, and killed cells of bacteria of the genus Bifidobacterium.

4. The composition according to any one of claims 1 - 3, wherein the anionic surfactant contains at least one selected from the group consisting of metal stearates, and the nonionic surfactant having an HLB value of less than 7 contains at least one selected from the group consisting of sucrose fatty acid esters having an HLB value of less than 7 and glycerine fatty acid esters having an HLB value of less than 7.

5. The composition according to any one of claims 1 to 4, wherein the bacterial powder is contained in an amount of 0.5 to 10 mass% based on the total mass of the composition.

6. The composition according to any one of claims 1 to 5, further comprising an additive.

7. A method for producing the composition according to any one of claims 1 to 6, comprising:
mixing a bacterial powder, a fatty oil, and at least one selected from the group consisting of a microscopic powder and a surfactant in an arbitrary order; or
mixing a suspension containing a bacterial powder and a fatty oil with at least one selected from the group consisting of a microscopic powder and a surfactant.

8. Use of at least one selected from the group consisting of a microscopic powder and a surfactant as an anti-caking agent for a bacterial powder in a composition containing a fatty oil, wherein the microscopic powder contains at least one selected from the group consisting of microcrystalline cellulose and tricalcium phosphate,
wherein the fatty oil is contained in an amount of 90 mass% or more,
and wherein the surfactant contains at least one selected from the group consisting of an anionic surfactant and a nonionic surfactant having an HLB value of less than 7.

## Patentansprüche

1. Zusammensetzung, umfassend:
ein bakterielles Pulver;
ein fettes Öl; und
mindestens eines ausgewählt aus der Gruppe bestehend aus einem mikroskopischen Pulver und einem Tensid, wobei das mikroskopische Pulver mindestens eines ausgewählt aus der Gruppe bestehend aus mikrokristalliner Cellulose und Tricalciumphosphat enthält,
wobei das fette Öl in einer Menge von 90 Massen-% oder mehr, basierend auf der Gesamtmasse der Zusammensetzung, enthalten ist,
und wobei das Tensid mindestens eines aus der Gruppe bestehend aus einem anionischen Tensid und einem nichtionischen Tensid mit einem HLB-Wert von weniger als 7 enthält.

2. Zusammensetzung gemäß Anspruch 1, die eine Suspension ist.

3. Zusammensetzung gemäß Anspruch 1 oder 2, wobei das bakterielle Pulver mindestens eines ausgewählt aus der Gruppe bestehend aus lebenden Zellen von Milchsäurebakterien, abgetöteten Zellen von Milchsäurebakterien, lebenden Zellen von Bakterien der Gattung Bifidobacterium und abgetöteten Zellen von Bakterien der Gattung Bifidobacterium enthält.

4. Zusammensetzung gemäß irgendeinem der Ansprüche 1 bis 3, wobei das anionische Tensid mindestens eines, ausgewählt aus der Gruppe bestehend aus Metallstearaten, enthält, und das nichtionische Tensid mit einem HLB-Wert von weniger als 7 mindestens eines, ausgewählt aus der Gruppe bestehend aus Saccharosefettsäureestern mit einem HLB-Wert von weniger als 7 und Glycerinfettsäureestern mit einem HLB-Wert von weniger als 7, enthält.

5. Zusammensetzung gemäß irgendeinem der Ansprüche 1 bis 4, wobei das bakterielle Pulver in einer Menge von 0,5 bis 10 Massen-%, basierend auf der Gesamtmasse der Zusammensetzung, enthalten ist.

6. Zusammensetzung gemäß irgendeinem der Ansprüche 1 bis 5, weiter umfassend einen Zusatzstoff.

7. Verfahren zur Herstellung der Zusammensetzung gemäß irgendeinem der Ansprüche 1 bis 6, umfassend:
Mischen eines bakteriellen Pulvers, eines fetten Öls und mindestens eines ausgewählt aus der Gruppe bestehend aus einem mikroskopischen Pulver und einem Tensid in einer beliebigen Reihenfolge; oder
Mischen einer Suspension, die ein bakterielles Pulver und ein fettes Öl enthält, mit mindestens einem, ausgewählt aus der Gruppe, die aus einem mikroskopischen Pulver und einem Tensid besteht.

8. Verwendung von mindestens einem ausgewählt aus der Gruppe bestehend aus einem mikroskopischen Pulver und einem Tensid als Antibackmittel für ein bakterielles Pulver in einer Zusammensetzung enthaltend ein fettes Öl, wobei das mikroskopische Pulver mindestens eines ausgewählt aus der Gruppe bestehend aus mikrokristalliner Cellulose und Tricalciumphosphat enthält,
wobei das fette Öl in einer Menge von 90 Massen-% oder mehr enthalten ist,
und wobei das Tensid mindestens eines, ausgewählt aus der Gruppe, bestehend aus einem anionischen Tensid und einem nichtionischen Tensid mit einem HLB-Wert von weniger als 7, enthält.

## Revendications

1. Composition comprenant :
une poudre bactérienne ;
une huile grasse ; et
au moins un élément choisi dans le groupe constitué par une poudre microscopique et un tensioactif, dans laquelle la poudre microscopique contient au moins un élément sélectionné dans le groupe constitué par cellulose microcristalline et phosphate tricalcique,
dans laquelle l'huile grasse est contenue en une quantité de 90 % en masse ou plus, sur la base de la masse totale de la composition ;
et dans laquelle le tensioactif contient au moins un élément sélectionné dans le groupe constitué par un tensioactif anionique et un tensioactif non ionique présentant une valeur HLB inférieure à 7.

2. Composition selon la revendication 1, qui est une suspension.

3. Composition selon la revendication 1 ou la revendication 2, dans laquelle la poudre bactérienne contient au moins un élément sélectionné dans le groupe constitué par des cellules vivantes de bactéries lactiques, des cellules tuées de bactéries lactiques, des cellules vivantes de bactéries du genre Bifidobacterium, et des cellules tuées de bactéries du genre Bifidobacterium.

4. Composition selon l'une quelconque des revendications 1 à 3, dans laquelle le tensioactif anionique contient au moins un élément sélectionné dans le groupe constitué par des stéarates métalliques, et le tensioactif non ionique présentant une valeur HLB inférieure à 7 contient au moins un élément sélectionné dans le groupe constitué par des esters d'acides gras de saccharose présentant une valeur HLB inférieure à 7 et des esters d'acides gras de glycérine présentant une valeur HLB inférieure à 7.

5. Composition selon l'une quelconque des revendications 1 à 4, dans laquelle la poudre bactérienne est contenue en une quantité de 0,5 à 10 % en masse sur la base de la masse totale de la composition.

6. Composition selon l'une quelconque des revendications 1 à 5, comprenant en outre un additif.

7. Procédé de production de la composition selon l'une quelconque des revendications 1 à 6, comprenant :
le mélange d'une poudre bactérienne, d'une huile grasse, et d'au moins un élément sélectionné dans le groupe constitué par une poudre microscopique et un tensioactif dans un ordre arbitraire ; ou
le mélange d'une suspension contenant une poudre bactérienne et une huile grasse avec au moins un élément sélectionné dans le groupe constitué par une poudre microscopique et un tensioactif.

8. Utilisation d'au moins un élément choisi dans le groupe constitué par une poudre microscopique et un tensioactif comme agent anti-agglomérant pour une poudre bactérienne dans une composition contenant une huile grasse, dans laquelle la poudre microscopique contient au moins un élément sélectionné dans le groupe constitué par cellulose microcristalline et phosphate tricalcique,
dans laquelle l'huile grasse est contenue en une quantité de 90 % en masse ou plus,
et dans laquelle le tensioactif contient au moins un élément sélectionné dans le groupe constitué par un tensioactif anionique et un tensioactif non ionique présentant une valeur HLB inférieure à 7.
